(19) Europäisches Patentamt
European Patent Office
Office européen des brevets

(11) Veröffentlichungsnummer: **0 006 170**
**B1**

(12) # EUROPÄISCHE PATENTSCHRIFT

(45) Veröffentlichungstag der Patentschrift: **02.06.82**

(51) Int. Cl.³: **C 07 C 49/403,** C 07 C 49/417, C 07 C 45/65

(21) Anmeldenummer: **79101730.4**

(22) Anmeldetag: **01.06.79**

(54) **Verfahren zur Herstellung von Cyclohexan-1.3-dionen aus Delta-Ketocarbonsäureestern.**

(30) Priorität: **08.06.78 DE 2825170**

(43) Veröffentlichungstag der Anmeldung: **09.01.80 Patentblatt 80/1**

(45) Bekanntmachung des Hinweises auf die Patenterteilung: **02.06.82 Patentblatt 82/22**

(84) Benannte Vertragsstaaten: **BE CH DE FR GB IT NL**

(56) Entgegenhaltungen:
**DE-A-2 448 677**
**DE-A-2 749 437**
**US-A-3 932 511**

(73) Patentinhaber: **HOECHST AKTIENGESELLSCHAFT, Postfach 80 03 20, D-6230 Frankfurt/Main 80 (DE)**

(72) Erfinder: **Müller, Werner Heinrich, Dr., Taunusblick 5, D-6239 Eppstein/Taunus (DE)**
Erfinder: **Mack, Karl Ernst, Dr., Kirchplatz 14, D-6233 Kelkheim (Taunus) (DE)**
Erfinder: **Hey, Hansjörg, Dr., Auwald 8, D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 006 170 B1

Verfahren zur Herstellung von Cyclohexan-1.3-dionen aus δ-Ketocarbonsäureestern

Die Erfindung betrifft ein Verfahren zur Herstellung von Cyclohexan-1.3-dionen durch Umsetzung von δ-Ketocarbonsäureestern an Katalysatoren.

Cyclohexan-1.3-dione sind begehrte Ausgangsmaterialien vieler Synthesen. Insbesondere können sie durch Dehydrierung in technisch wichtige Resorcine übergeführt werden.

Es ist bekannt, daß Cyclohexan-1.3-dione durch Cyclisierung von δ-Ketocarbonsäureestern (DE-OS 2 245 270) oder δ-Enollactonen (DE-OS 2 261 751) hergestellt werden können. Die beiden Verfahren werden in der Flüssigphase in Gegenwart starker Basen durchgeführt und haben den Nachteil, daß beträchtliche Mengen wertloser, anorganischer Salze anfallen und daß erhebliche Mengen an Lösungsmittel erforderlich sind.

Weiterhin ist bekannt, daß Cyclohexan-1.3-dione durch katalytische Umsetzung von δ-Ketocarbonsäuren (DE-OS 2 448 677) oder von δ-Ketocarbonsäureestern (US-PS 3 932 511) in der Gasphase zugänglich sind. Beim letztgenannten Verfahren werden als Katalysatoren Materialien verwendet, die thermisch stabil sind, eine innere Oberfläche von 100—1500 $m^2/g$ besitzen und die Cyclisierungsreaktion katalysieren. Bevorzugt werden Kohle, Graphit und Oxide der Erdalkalimetalle, im besonderen Kohle verwendet.

Die beiden zuletzt genannten Verfahren haben jedoch den Nachteil, daß hier die Umsätze der Ausgangsverbindungen, insbesondere des δ-Ketohexansäureesters, relativ gering sind, so daß die Isolierung der thermisch meist instabilen Cyclohexan-1.3-dione nur unter relativ hohem Verlust möglich ist.

Es wurde nun ein Verfahren zur Herstellung von Cyclohexan-1.3-dionen der allgemeinen Formel

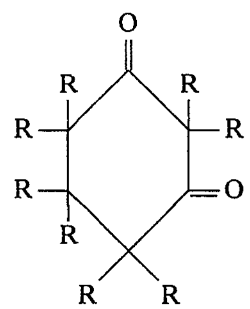

in der die einzelnen Reste R gleich oder verschieden und unabhängig voneinander Wasserstoff, Alkyl, Cycloalkyl oder Aryl mit einer Gesamtzahl von bis zu 12 Kohlenstoffatomen sein können, durch Umsetzung von δ-Ketocarbonsäureestern der allgemeinen Formel

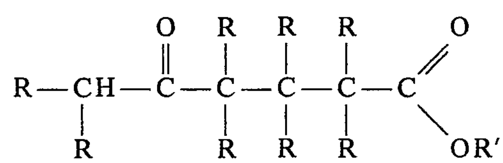

wobei R die obige Bedeutung hat und R′ ein Alkyl-, Cycloalkyl- oder Aryl-Rest mit bis zu 9 Kohlenstoffatomen ist, in der Gasphase bei Temperaturen von 250 bis 500°C an einem Katalysator gefunden, das dadurch gekennzeichnet ist, daß der Katalysator aus einem Trägermaterial besteht, auf das mindestens ein Element der Gruppe IIIB oder IVB des Periodensystems in Form einer Verbindung aufgebracht ist. Bei diesem Verfahren ist der Umsatz an δ-Ketocarbonsäureestern — insbesondere an δ-Ketohexansäureester — wesentlich höher als bei dem oben genannten Verfahren gemäß US-PS 3 932 511. Zwar werden auch bei dem bereits erwähnten Verfahren gemäß DE-OS 2 448 677 Oxide von Elementen der dritten oder vierten Nebengruppe eingesetzt, jedoch geht dieses Verfahren von der freien 5-Ketosäure statt vom Ester aus und legt daher das vorliegende Verfahren keineswegs nahe. Der Reaktionsdruck ist nicht kritisch; im allgemeinen arbeitet man bei Normaldruck, jedoch sind auch Über- oder Unterdrücke geeignet. Als besonders geeigneter Druckbereich ist 0.1 bis 10 bar anzusehen. Die Reaktionstemperatur beträgt vorzugsweise 300 bis 400°C.

Als Reste R im δ-Ketocarbonsäureester sind verzweigte oder geradkettige Alkylreste geeignet, beispielsweise Methyl, Ethyl, Propyl, Butyl, Pentyl, Hexyl, Octyl, Decyl und Dodecyl. Als Cycloalkylgruppen sind beispielsweise Cyclopentyl, Cyclohexyl, Cyclodecyl oder Cyclododecyl geeignet. Bevorzugt sind Alkylreste oder Cycloalkylreste mit bis zu 6 Kohlenstoffatomen. Als Arylreste kommen die Phenyl- und die Naphthylgruppe in Frage. Vorteilhaft ist es, wenn jeweils mindestens einer der Reste R an ein und demselben C-Atom Wasserstoff ist, vorzugsweise sind 6 der 8 Reste R Wasserstoff, besonders bevorzugt ist der Fall, daß alle 8 Reste R Wasserstoff bedeuten.

Der Rest R' im δ-Ketocarbonsäureester ist vorzugsweise eine geradkettige Alkylgruppe oder ein Aralkylrest wie Benzyl, Tolyl, Phenylethyl, Phenylpropyl. Besonders bevorzugt ist Methyl, Ethyl, n-Propyl oder n-Butyl.

Von den Elementen der Gruppen IIIB und IVB, d. h. Scandium, Yttrium, Lanthan und den Lanthaniden, Titan, Zirkon, Hafnium und den Actiniden, sind Lanthan, die Lanthaniden, Zirkon und Thorium bevorzugt. Besonders bevorzugt ist Thorium.

Es können mehrere Elemente der beiden genannten Gruppen des Periodensystems aufgebracht werden. Vorzugsweise wird jedoch nur ein Element verwendet.

Neben den genannten Elementen zeigen allerdings auch Rubidium, Strontium, Aluminium, Zinn, Blei, Wismut, Molybdän, Mangan, Rhenium, Cobalt, Kupfer, Zink und Cadmium gelegentlich eine katalytische Wirkung.

Der Anteil der Elemente der Gruppen IIIB und IVB, die auf das Trägermaterial aufgebracht werden, am Katalysatorgewicht beträgt im allgemeinen 0.05 bis 10 Gew.-% vorzugsweise 0.1 bis 5 Gew.-%.

Das Trägermaterial kann amorph oder kristallin sein und hat vorzugsweise eine Innenoberfläche von 50 bis 1500 $m^2/g$. Geeignet sind beispielsweise Aluminiumoxid, Titandioxid, Zirkondioxid, Alumosilikate, Spinelle, Zeolithe, Aluminiumoxid-Chromoxid oder Kohle. Vorzugsweise verwendet man Kohle.

Ein besonders bevorzugter Katalysator ist somit Kohle, imprägniert mit einer Thoriumverbindung.

Durch Verwendung des erfindungsgemäßen Katalysators gelingt es, bei Umsätzen von 45 bis 50% an δ-Ketocarbonsäureester — insbesondere an δ-Ketohexansäureester — die Cyclohexan-1.3-dione in über 90%iger Selektivität zu erhalten. Die anfallende Reaktionsmischung enthält dabei über 35 Gew.-% Cyclohexan-1.3-dion. Nahezu 80 Gew.-% des Cyclohexan-1.3-dions kristallisiert aus und kann einer weiteren Reinigung zugeführt werden.

Die Herstellung des Katalysators erfolgt durch Aufbringen einer Verbindung eines Elements der Gruppe IIIB oder IVB (oder mehrerer solcher Verbindungen) auf das Trägermaterial nach einem der üblichen Tränkeverfahren. Geeignete Metallverbindungen sind z. B. solche, die sich von Sauerstoff enthaltenden Säuren ableiten, wie Nitrate, Sulfate, Acetate, Oxalate, weiterhin Halogenide, sowie von C—H-aciden organischen Verbindungen (wie beispielsweise Acetylaceton) abgeleitete Verbindungen.

Das Anion ist jedoch nicht kritisch.

Für den Tränkvorgang können Wasser oder polare organische Lösungsmittel, wie beispielsweise Alkohole, Ketone oder Nitrile verwendet werden; bevorzugt ist Wasser. Schwer wasserlösliche Metallsalze können häufig durch komplexbildende Zusatzstoffe in wäßrige Lösung gebracht werden. So ist beispielsweise Thoriumoxalat im Gemisch mit Ammoniumoxalat gut wasserlöslich. In den Fällen, wo solche Zusatzstoffe verwendet werden, ist es zweckmäßig, sie nach dem Trocknen der Katalysatoren durch Calcinieren zu zerstören. Je nach Art des Trägermaterials und des Zusatzstoffes sind hierfür Temperaturen von 400 bis 700° C in Stickstoffatmosphäre geeignet.

Die Katalysatoren können, falls notwendig, mehrfach regeneriert werden. Dies geschieht im allgemeinen durch Überleiten eines Gasgemisches, bestehend aus Sauerstoff und einem weiteren Gas, wie beispielsweise Stickstoff, Kohlendioxid, Wasserdampf oder einem Edelgas über den Katalysator. Die Temperatur wird dabei je nach Trägermaterial bei 150 bis 600° C gehalten, vorzugsweise bei 250 bis 450° C. Der Sauerstoffgehalt des Gasgemisches beträgt im allgemeinen 0.5 bis 10 Vol.-%, vorzugsweise 1 bis 5 Vol.-%. Besonders geeignet ist ein Gemisch aus Luft und Stickstoff. Nach mehrstündiger Behandlung des Katalysators mit dem erwähnten Gasgemisch hat er seine ursprüngliche Aktivität im wesentlichen wiedererlangt.

Die Durchführung des erfindungsgemäßen Verfahren erfolgt im allgemeinen durch Überleiten der gasförmigen δ-Ketocarbonsäureester im verdünnten Zustand über den Katalysator. Das molare Verhältnis von Verdünnungsmittel zu Ausgangsverbindung ist dabei 1 : 1 bis 50 : 1, vorzugsweise 3 : 1 bis 20 : 1.

Als Verdünnungsmittel werden im allgemeinen Gase wie Stickstoff, Wasserstoff, Edelgas, Kohlendioxid, Wasserdampf, Gemische dieser Gase in beliebigen Proportionen, weiterhin verdampfte organische Lösungsmittel oder deren Gemische mit Stickstoff, Wasserstoff, Edelgas, Kohlendioxid oder Wasserdampf (Volumenanteil des organischen Lösungsmittels 1 bis 50%) und weiterhin Gemische aus Stickstoff, Edelgas, Kohlendioxid oder Wasserdampf mit Luft (Volumenanteil der Luft 1 bis 30%) verwendet. Geeignete organische Lösungsmittel sind dabei cyclische oder acyclische Ether (wie beispielsweise Tetrahydrofuran, 1.4-Dioxan, Diisopropylether), Ether der Ethylenglykole (wie beispielsweise Dimethyldiethylenglykol, Diethyldiethylenglykol, Methylbutyldiethylenglykol, Ethylbutyldiethylenglykol, Dibutyldiethylenglykol, Methylethyltriethylenglykol oder Dibutyltriethylenglykol), weiterhin Ester organische Carbonsäuren (wie beispielsweise Ethylacetat, Butan-1.4-diacetat und Dimethylsuccinat), sowie halogenierte Kohlenwasserstoffe (wie beispielsweise Chloroform, Tetrachlorkohlenstoff und 1.2-Dichlormethan). Vorzugsweise wird als Verdünnungsmittel ein Gemisch aus Stickstoff und Wasserstoff (Molverhältnis 1 : 1) oder ein Gemisch aus Stickstoff und einem Ether der Ethylenglykole (Molverhältnis 1 : 1) verwendet.

Folgende Arbeitsweise hat sich zur Durchführung des erfindungsgemäßen Verfahrens als besonders günstig erwiesen: Die Umsetzung des δ-Ketocarbonsäureesters erfolgt in einem senkrecht

stehenden Reaktor; der Katalysator befindet sich in dessen mittleren Teil. Die Ausgangsverbindung wird — gegebenenfalls durch ein organisches Lösungsmittel verdünnt — in einen Verdampfer gegeben, dann mit einem Gemisch aus Wasserstoff und Stickstoff gemischt und über den Katalysator geleitet. Da der Druck für die Reaktion keine kritische Größe ist, wird bei Normaldruck gearbeitet. Das Reaktionsgemisch wird am Reaktorende zweistufig kondensiert, so daß die Hauptmenge des zwangsläufig anfallenden Alkohols R' OH nicht zusammen mit dem Cyclohexan-1.3-dion in der ersten Stufe kondensiert, sondern erst in der zweiten Stufe. Das Cyclohexan-1.3-dion in der ersten Stufe kristallisiert beim weiteren Abkühlen aus.

## Vergleichsbeispiel

Ein senkrecht stehendes Reaktionsrohr, dessen Mittelteil mit 50 ml Trägermaterial gefüllt ist, wird auf 350°C erhitzt. Von oben her wird stündlich ein Gasgemisch aus 10 Nl Stickstoff, 10 Nl Wasserstoff und 1.5 NL $\delta$-Ketohexansäuremethylester in den Reaktor geleitet. Als Trägermaterialien werden der Reihe nach gekörnte Aktiv-Kohle, gekörntes Aluminiumoxid, bzw. Strangpreßlinge der Oxide von Aluminium, Silicium, Molybdän, Calcium, Titan, Zirkon, Lanthan und Thorium verwendet. Das aus dem Reaktor austretende Produkt wird kondensiert und durch Gaschromatographie analysiert.

In Tabelle 1 sind die Umsätze an $\delta$-Ketohexansäuremethylester und die Selektivitäten der Bildung von Cyclohexan-1.3-dion (CD) während einer Versuchsdauer von 40 Stunden angegeben.

Tabelle 1

| Trägermaterial | | % Umsatz | % Selektivität |
|---|---|---|---|
| Kohle | gekörnt | 12 | 82 |
| $Al_2O_3$ | gekörnt | 10 | 73 |
| $Al_2O_3$ | Strangpreßlinge | 8 | 62 |
| $SiO_2$ | Strangpreßlinge | 7 | 58 |
| $MoO_3$ | Strangpreßlinge | 11 | 66 |
| CaO | Strangpreßlinge | 9 | 68 |
| $TiO_2$ | Strangpreßlinge | 10 | 74 |
| $ZrO_2$ | Strangpreßlinge | 8 | 73 |
| $La_2O_3$ | Strangpreßlinge | 9 | 65 |
| $ThO_2$ | Strangpreßlinge | 11 | 68 |

Dieses Beispiel zeigt, daß mit unimprägnierten Trägermaterialien nur relativ geringe Umsätze an $\delta$-Ketohexansäuremethylester zu Cyclohexan-1.3-dion erreicht werden.

## Beispiel 1

Gekörntes Aluminiumoxid (Oberfläche ca. 80 $m^2/g$) bzw. gekörnte Kohle (Oberfläche ca. 1000 $m^2/g$) werden mit wäßrigen Lösungen der Nitrate von Lanthan, Cer oder Zirkon bzw. einem Gemisch aus Thoriumoxalat/Ammoniumoxalat (Gewichtsverhältnis 1 : 2) getränkt, bei 120°C 6 Stunden lang getrocknet und danach bei 420°C während 6 Stunden in einer Stickstoffatmosphäre calciniert. Auf das Trägermaterial wird jeweils eine solche Menge der genannten Verbindungen aufgebracht, daß der Gehalt an aktiven Elementen (La, Ce, Zr, Th) im Katalysator 1 Gew.-% beträgt. Die Ergebnisse der Umsetzung von $\delta$-Ketohexansäuremethylester zu Cyclohexan-1.3-dion (CD) unter denselben Bedingungen wie im Vergleichsbeispiel, aber während 280 Stunden Betriebsdauer, sind in Tabelle 2 zusammengefaßt.

Tabelle 2

| Element | Träger: $Al_2O_3$ | | Träger: Kohle | |
|---|---|---|---|---|
| | Umsatz | Selektivität | Umsatz | Selektivität |
| | % | % CD | % | % CD |
| La | 28 | 89 | 32 | 92 |
| Ce | 27 | 92 | 29 | 93 |
| Zr | 31 | 90 | 36 | 91 |
| Th | 37 | 94 | 48 | 95 |

Dieses Beispiel zeigt, daß durch Zusatz der Elemente Lanthan, Cer, Zirkon, besonders aber Thorium, zu den Trägermaterialien $Al_2O_3$ und Kohle erhebliche Umsatzsteigerungen und auch Selektivitätsverbesserungen erreicht werden, und dies sogar über längere Zeit.

Beispiel 2

Es wird ein Katalysator hergestellt, indem man gekörnte Kohle mit einer wäßrigen Lösung von Thoriumnitrat tränkt und wie in Beispiel 1 trocknet und calciniert. Der fertige Katalysator enthält 2 Gew.-% Thorium und wird zur Umsetzung von $\delta$-Ketohexansäuremethylester zu Cyclohexan-1.3-dion unter denselben Bedingungen wie im Vergleichsbeispiel benutzt.

Nach 300, 600 bzw. 900 Stunden Betriebsdauer wird der Katalysator regeneriert, indem er im Reaktor bei einer Temperatur von 380° C während 5 Stunden mit einem Gasgemisch aus Stickstoff und Luft (1 Vol.-% Sauerstoff) behandelt wird. Anschließend wird die Umsetzung fortgesetzt. Es werden folgende Umsätze und Selektivitäten für CD erzielt:

| | Betriebsdauer (Stunden) | | | | | | | | | | | |
|---|---|---|---|---|---|---|---|---|---|---|---|---|
| | 10 | 300 | | 310 | 600 | | 610 | 900 | | 910 | 1200 |
| % Umsatz | 52 | 45 | Regenerierung | 50 | 44 | Regenerierung | 48 | 44 | Regenerierung | 49 | 45 |
| % Selektivität CD | 88 | 92 | | 90 | 95 | | 90 | 93 | | 90 | 93 |

**Patentansprüche**

1. Verfahren zur Herstellung von Cyclohexan-1.3-dionen der allgemeinen Formel

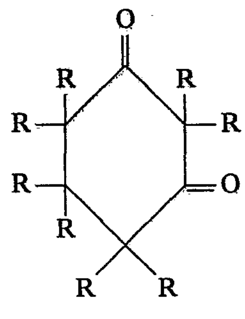

in der die einzelnen Reste R gleich oder verschieden und unabhängig voneinander Wasserstoff, Alkyl, Cycloalkyl oder Aryl mit einer Gesamtzahl von bis zu 12 Kohlenstoffatomen sein können, durch Umsetzung von $\delta$-Ketocarbonsäureestern der allgemeinen Formel

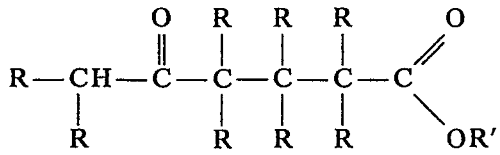

wobei R die obige Bedeutung hat und R' ein Alkyl-, Cycloalkyl- oder Aryl-Rest mit bis zu 9 Kohlenstoffatomen ist, in der Gasphase bei Temperaturen von 250 bis 500°C an einem Katalysator, dadurch gekennzeichnet, daß der Katalysator aus einem Trägermaterial besteht, auf das mindestens ein Element der Gruppe IIIB oder IVB des Periodensystems in Form einer Verbindung aufgebracht ist.

2. Verfahren nach Anspruch 1, dadurch gekennzeichnet, daß auf das Trägermaterial eine Verbindung der Elemente Lanthan, der Lanthaniden, Zirkon oder Thorium aufgebracht ist.

3. Verfahren nach Anspruch 1, dadurch gekennzeichnet, daß auf das Trägermaterial eine Thoriumverbindung aufgebracht ist.

4. Verfahren nach einem der Ansprüche 1 bis 3, dadurch gekennzeichnet, daß der Gewichtsanteil der auf das Trägermaterial aufgebrachten Elemente 0.05 bis 10% des Katalysatorgewichts beträgt.

5. Verfahren nach einem der Ansprüche 1 bis 4, dadurch gekennzeichnet, daß das Trägermaterial aus Kohle besteht.

6. Verfahren nach einem der Ansprüche 1 bis 5, dadurch gekennzeichnet, daß der δ-Ketocarbonsäureester mit der 3- bis 20fachen Menge eines äquimolaren Gemisches aus Stickstoff und Wasserstoff oder aus Stickstoff und einem Ethylenglykol-Ether verdünnt wird.

7. Verfahren zur Regenerierung eines Katalysators zur Herstellung von Cyclohexan-1.3-dionen aus δ-Ketocarbonsäureestern, der aus einem Trägermaterial besteht, auf das mindestens ein Element der Gruppe IIIB oder IVB des Periodensystems in Form einer Verbindung aufgebracht ist, dadurch gekennzeichnet, daß man über den Katalysator bei Temperaturen von 150 bis 600°C ein Gemisch aus Sauerstoff und Stickstoff, Kohlendioxid, Wasserdampf oder einem Edelgas leitet.

8. Verfahren nach Anspruch 7, dadurch gekennzeichnet, daß als Trägermaterial Kohle verwandt wird.

## Claims

1. Process for the preparation of cyclohexane-1,3-diones of the general formula

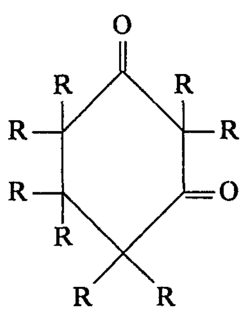

in which the individual radicals R may be identical or different and independently of one another may represent hydrogen, alkyl, cycloalkyl or aryl with the sum of all carbon atoms of the radicals taken together being up to 12, by reacting δ-ketocarboxylic acid esters of the general formula

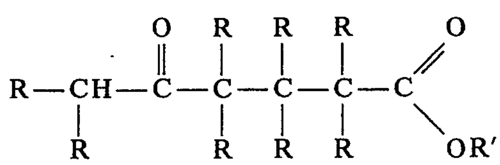

R being defined as above and R' representing alkyl, cycloalkyl or aryl of up to 9 carbon atoms, in the gaseous phase at a temperature of from 250 to 500°C at a catalyst, characterized in that the catalyst comprises a carrier material onto which at least one element of groups III B or IVB of the Periodic System has been applied in the form of a compound.

2. Process as claimed in claim 1, wherein a compound of the elements lanthanum, the lanthanides, zirconium or thorium has been applied onto the carrier material.

3. Process as claimed in claim 1, wherein a thorium compound has been applied onto the carrier material.

4. Process as claimed in any one of claims 1 to 3, wherein the proportion by weight of the elements applied onto the carrier material is from 0.05 to 10% of the catalyst weight.

5. Process as claimed in any one of claims 1 to 4, wherein the carrier material consists of carbon.

6. Process as claimed in any one of claims 1 to 5, which comprises diluting the δ-ketocarboxylic acid ester with 3 to 20 times the amount of an equimolar mixture of nitrogen and hydrogen or of nitrogen and an ethylene-glycol ether.

7. Process for regenerating a catalyst for the preparation of cyclohexane-1,3-diones from δ-ketocarboxylic acid esters, which consists of a carrier material onto which at least one element of groups IIIB or IVB of the Periodic System has been applied in the form of a compound, characterized by passing a mixture of oxygen and nitrogen, carbon dioxide, steam or a noble gas over the catalyst at a temperature of from 150 to 600° C.

8. Process as claimed in claim 7, wherein carbon is used as carrier material.

**Revendications**

1. Procédé de préparation de cyclohexane-diones-1,3-répondant à la formule

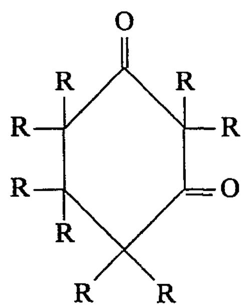

dans laquelle les divers symboles R représentent chacun, indépendamment les uns des autres, l'hydrogène, un alkyle, un cyclo-alkyle ou un aryle avec un nombre total d'atomes de carbone d'au plus 12, par réaction d'esters d'acides δ-oxo-carboxyliques répondant à la formule générale:

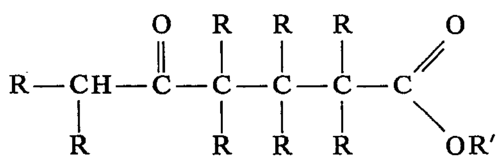

dans laquelle les R ont les significations qui viennent d'être données et R' représente un radical alkyle, cycloalkyle ou aryle contenant au plus 9 atomes de carbone, en phase gazeuse, à des températures de 250 à 500° C, en présence d'un catalyseur, procédé caractérisé en ce que le catalyseur est constitué d'une matière support sur laquelle est déposé au moins un élément du groupe IIIB ou IVB de la classification périodique, sous la forme d'un composé.

2. Procédé selon la revendication 1, caractérisé en ce qu'on applique, sur la matière support, un composé d'un des éléments suivants: lanthane, lanthanides, zirconium et thorium.

3. Procédé selon la revendication 1, caractérisé en ce qu'on applique un composé du thorium sur la matière support.

4. Procédé selon l'une quelconque des revendications 1 à 3, caractérisé en ce que la proportion pondérale des éléments appliqués sur la matière support est comprise entre 0,05 et 10% par rapport au poids du catalyseur.

5. Procédé selon l'une quelconque des revendications 1 à 4, caractérisé en ce que la matière support est constituée de charbon.

6. Procédé selon l'une quelconque des revendications 1 à 5, caractérisé en ce que l'ester d'acide δ-oxocarboxylique est dilué avec de 3 à 20 fois sa quantité d'un mélange équimolaire d'azote et d'hydrogène, ou d'azote et d'un éther d'éthylène-glykol.

7. Procédé pour régénérer un catalyseur devant servir à la préparation de cyclohexane-diones-1,3 à partir d'esters d'acides δ-oxo-carboxyliques, constitué d'une matière support sur laquelle a été déposé au moins un élément du groupe IIIB ou IVB de la classification périodique, sous la forme d'un composé, procédé caractérisé en ce qu'on fait passer sur le catalyseur, à des températures de 150 à 600° C, un mélange constitué d'oxygène et d'azote, de dioxyde de carbone, de vapeur d'eau ou d'un gaz rare.

8. Procédé selon la revendication 7, caractérisé en ce qu'on utilise du charbon comme matière support.